# EUROPÄISCHE PATENTSCHRIFT

(11) **EP 0 502 876 B1**
(45) Veröffentlichungstag und Bekanntmachung des Hinweises auf die Patenterteilung: **08.03.1995**
(21) Anmeldenummer: 90917037.5
(22) Anmeldetag: 22.11.1990
(51) Int. Cl.: C12N 9/48, A61K 35/62

(54) **NEUE BLUTGERINNSELAUFLÖSENDE PROTEINE UND IHRE HERSTELLUNG AUS DEM BLUTEGEL HIRUDO MEDICINALIS**
NEW BLOOD-CLOT-DISSOLVING PROTEINS, AND THEIR PREPARATION FROM THE LEECH HIRUDO MEDICINALIS
NOUVELLES PROTEINES DISSOLVANT LES CAILLOTS SANGUINS, ET LEUR PREPARATION A PARTIR DE LA SANGSUE HIRUDO MEDICINALIS

(30) Priorität: 01.12.1989 DE 3939801
(43) Veröffentlichungstag der Anmeldung: 16.09.1992
(73) Patentinhaber: BASF Aktiengesellschaft, 67063 Ludwigshafen (DE)
(72) Erfinder: MEYER, Thomas, D-6800 Mannheim 1 (DE); FRIEDRICH, Thomas, D-6100 Darmstadt (DE); KOERWER, Wolfgang, D-6718 Gruenstadt (DE); STRUBE, Karl-Hermann, D-6720 Speyer (DE); BIALOJAN, Siegfried, D-6836 Oftersheim (DE)
(86) Internationale Anmeldenummer: EP9001996
(87) Internationale Veröffentlichungsnummer: WO9108233

(56) Entgegenhaltungen:
- EP-A- 0 146 785
- EP-A- 0 158 986
- EP-A- 0 209 061
- US-A- 4 588 587
- Dialog information services file 155, Medline NLM accession number 90366617 "Hydrolysis of the iso-oeotide epsilon-(gamma-glutamyl)-lysine by destabilase from the medioinal leech Hirudo medicinalis", Baslova IP et al, Biokhimiia (USSR) May 1990, 55 (5) p 771-5
- Dialog information system file 155 Medline NLM accession number 90335324 "Kinetics of L-gamma-Glu-pNA hydrolysis by destabilase, the enzyme from the medicinal leech Hirudo medicinalis, Baskova IP et al Biokhimiia (USSR) Apr 1990, 55 (4) p674-9
- Dialog information services file 155 Medline NLM accession number85200114, "Destabilase: an enzyme of medicinal leech salivary gland secretion hydrolyzes the isopeptide bonds in stabilized fibrin ", Boskova IP et al, Biokhimiia Mar 1985, 50 (3) p 424-31

## Beschreibung

Die vorliegende Erfindung betrifft neue Proteine und deren Herstellung.

Es sind bereits eine Reihe von Proteinen - wie tPA, Urokinase und Streptokinase - bekannnt, die Blutgerinnsel auflösen können. Ihre Wirkung besteht darin, daß sie das im Blutgerinnsel vorhandene Fibrin durch Spaltung von Peptidketten depolymerisieren und damit löslich machen. Die Auflösung von Blutgerinnseln kann auch dadurch erfolgen, daß ε-(γ-Glutamyl)-Lysin-Bindungen gelöst werden. Solche Stoffe sind aus Hirudo medicinalis isoliert worden. Hierzu gehört die Destabilase (Biochemistry, USSR 50, 357 (1985)), die ein Molekulargewicht von 12.300 Da besitzt (XII Congress of the International Society on Thrombosis and Haemostasis, Abstract 1727 (1989)).

Gegenstand der Erfindung ist ein neues Protein, welches die im Sequenzprotokoll aufgeführten Sequenzen 1-12 enthält und ε-(γ-Glutamyl)-Lysin-Bindungen spaltet, sowie dessen Muteine und natürliche Varianten mit einer vergleichbaren Wirkung.

Als Muteine sind Proteine zu verstehen, die sich von dem neuen Protein durch Austausch, Deletion und/oder Addition von Aminosäuren oder Peptiden in der Proteinkette herleiten.

Als natürliche Varianten sind Proteine zu verstehen, die in blutsaugenden Tierarten vorkommen und bei einer ähnlichen Aminosäuresequenz die gleiche Wirkung zeigen.

Das neue Protein läßt sich aus Drüsenabsonderungen des Blutegels (Hirudo medicinalis) durch Chromatographie an S-Sepharose®, Concanavalin A- und Kupferchelatchromatographiesäulen isolieren. Aus den Drüsenabsonderungen von 500 g Blutegeln lassen sich 30 bis 200 Aktivitätseinheiten des Proteins isolieren.

Das neue Protein liegt in den Drüsenabsonderungen in Konzentrationen zwischen 1 - 100 »g/kg vor. Um das Protein für pharmazeutische Zwecke in größeren Mengen verfügbar zu machen, kann man bekannte gentechnische Methoden (vgl. Sambrook, T. et al: Molecular Cloning: A Laboratory Manual, Cold Spring Harbor Press, N.Y., 1989) heranziehen. Für diesen Zweck muß zunächst die genetische Information für das neue Protein identifiziert und die entsprechende Nukleinsäure isoliert werden. Dazu wird das reine Protein mit Dithiothreitol reduziert, dann wird Iodacetamid zur Derivatisierung der freien SH-Gruppen zugesetzt, und anschließend wird das so behandelte Protein mit Bromcyan oder Trypsin in kleine Peptide gespalten. Die Auftrennung der Peptide erfolgt über reversed phase-Chromatographie. Die gereinigten Peptide werden anschließend sequenziert.

Die Vorhandenen Peptidsequenzen erlauben nun durch die Synthese entsprechender Oligonukleotide eine eindeutige Identifizierung des Gens aus dem Genom oder aus entsprechenden cDNA-Bänken durch sequenzspezifische Filterhybridisierung. Die hierzu notwendigen Methoden der Herstellung von degenerierten Oligonukleotidsonden aus Proteinsequenzdaten, die Isolierung von mRNA und ihre Umschreibung in cDNA sowie die Herstellung und die Hybridisierung von cDNA-Bänken mit Oligonukleotidsonden sind Stand der Technik und im Lehrbuch "Molecular Cloning" Sambrook et al (1989) Kapitel 11, 7 und 8) beschrieben.

Die so erhaltene genetische Information für das Protein kann dann in verschiedenen Wirtszellen, wie eukaryotische Zellen, Hefen, Bacillus subtilis oder E. coli nach bekannten Methoden zur Expression gebracht und das Protein in größeren Mengen so erhalten werden. Auch die für die Expression heterologer Gene notwendigen Methoden sind Stand der Technik und werden in Sambrook et al. (1989), Kapitel 16 und 17 erläutert.

Auch die Muteine, werden vorzugsweise nach gentechnischen Methoden dargestellt.

Isoliert man das neue Protein aus Hirudo medicinals, so erhält man es in glykosylierter Form. Dasselbe gilt für die gentechnische Herstellung, wenn man zur Expression eukaryotische Zellen verwendet. Exprimiert man es in prokaryotischen Zellen, so erhält man es in nicht-glykosylierter Form. Beide Formen zeigen die gleiche Wirkung.

Das neue Protein ist eine Isopeptidase und kann durch seine enzymatische Aktivität die von dem aktivierten Faktor XIII quervernetzten Fibringerinnsel (Thrombus) auflösen. Diese Quervernetzung wird durch eine Isopeptidbindung zwischen Glutamat- und Lysinseitenketten gebildet. Die Spaltung erfolgt durch den Transfer eines y-Glutamylrestes auf eine andere Aminosäure, ein Dipeptid oder durch Hydrolyse. Durch die Hydrolyse dieser Isopeptidbindung wird die Wirkung von Faktor XIIIa aufgehoben. So werden durch Faktor XIIIa quervernetzte (gealterte) Gerinnsel verjüngt und so entweder direkt aufgelöst oder in Kombination mit Gewebeplasminogenaktivatoren (z.B. tPA) aufgelöst. So kann dieses Enzym in der Behandlung thrombotischer Zustände (z.B. Myocardinfarkt, tiefe Venenthrombose und Atherosklerose) in der akuten Therapie wie auch in der Vorbeugung eingesetzt werden.

Auch freigesetzte Tumorzellen verankern sich über eine ε-(γ-Glutamyl)-Lysin-Bindung in ihrem Zielgewebe und entziehen sich dem Immunsystem dann durch Fibrinummantelung. Das neue Enzym eignet sich daher auch zur Verhinderung der Bildung von Metastasen.

### Gewinnung des neuen Proteins

### Beispiel 1

### a) Gewinnung des Egelsekrets

500 g Hirudo medicinalis wurden bei 4°C im Kühlraum mit wenig Wasser in Portionen zu ca. 50 g gehalten. Die Tiere wurden 2 h in 150 mM NaCl, 10 mM Arginin und 20 mM Phosphatpuffer ph 7,4 bei Raumtemperatur stimuliert. Nach 2 Tagen wurden sie 1 h in 150 mM NaCl, 20 mM Phosphatpuffer pH 7,4 und 1 % Pilocarpin gebadet. Dabei sonderten sie große Mengen Schleim sowie eine enzymatische Aktivität ab, die das Farbsubstrat γ-Glutamyl-para-Nitroanilid umsetzt. 500 g Egel sonderten 30 bis 200 Units dieser enzymatischen Aktivität ab.

### b) Isolierung des Proteins aus dem Egelsekret

100 Units (vgl. a) wurden mit 5 l Äquilibrierungspuffer (20 mM Natriumphosphatpuffer pH 4,5, 10 mM NaCl, 0,01 % Tween® 80) verdünnt. Der pH wurde auf 4,5 korrigiert. Das Endvolumen betrug ca. 6 l. Das Volumen wurde mit einer Fließgeschwindigkeit von 5 ml/Minute auf 350 ml S-Sepharose® aufgetragen, mit 4 Säulenvolumen Äquilibrierungspuffer gewaschen und mit einem Salz-Gradienten von 1 l 20 mM Natriumphosphatpuffer pH 4,5, 10 mM NaCl, 0,01 % Tween® 80 nach 1 l 20 mM Natriumphosphatpuffer pH 4,5, 500 mM NaCl, 0,01 % Tween® 80 entwickelt. Die enzymatische Aktivität eluierte bei 50 bis 200 mM NaCl.

Die aktiven Fraktionen wurden gesammelt. Durch Verdünnung wurde der Puffer auf 20 mM Natriumacetat pH 7,0, 150 mM NaCl, 1 mM MnCl₂, 1 mM MgCl₂ und 1 mM CaCl₂ eingestellt. Diese Lösung wurde auf eine Concanavalin A-Säule (25 ml) gegeben und mit 4 Säulenvolumen des gleichen Puffers gewaschen. Die Elution erfolgte mit dem gleichen Puffer mit zusätzlich 10 mM Mannose oder 5 mM α-Methylmannosid.

Die aktiven Fraktionen wurden gesammelt und in einem Puffer so verdünnt, daß eine Endkonzentration von 20 mM Natriumphosphat pH 7,0, 500 mM Natriumchlorid, 0,01 % Tween® 80, erreicht wurde. Eine Kupferchelatsäule (2 ml Volumen) äquilibriert im gleichen Puffer, wurde beladen und mit 15 Säulenvolumen Äquilibrierungspuffer, gefolgt von 15 Säulenvolumen 20 mM Bernsteinsäure pH 7,0, 500 mM NaCl, 0,01 % Tween® 80 gewaschen. Danach wurde die Säule mit einem Gradienten nach 20 mM Bernsteinsäure pH 4,5, 500 mM NaCl, 0,01 % Tween 80 eluiert. Die Aktivität eluierte bei pH 6 bis 5,4.

### c) Reinigung des Proteins über Mono S-Chromatographie

Der Wertpeak der Cu-Chelat-Säule wurde konzentriert und auf 20 mM Na-Phosphat, 10 mM NaCl pH 5,0 (Puffer A) umgepuffert. Danach erfolgte der Auftrag auf eine Mono S FPLC-Säule (Pharmacia, Freiburg). Nach der Elution des nichtgebundenen Materials durch Spülen der Säule mit 10 Säulenvolumen Puffer A wurde die Säule mit einem kombinierten linearen pH- und Salzgradienten von 0 bis 66 % Puffer B (20 mM Natriumphosphat, 500 mM NaCl, pH 8,5) in 85 min eluiert. Unter diesen Bedingungen eluierte das neue Protein zwischen 10 und 30 % Puffer B von der Säule.

Die Elution wurde durch UV-Messing bei 280 nm bzw. Aktivitätsmessung von Aliquots der aufgetragenen Fraktionen verfolgt. Die Aktivität enthaltenden Fraktionen wurden vereinigt, entsalzt und zur Trockne aufkonzentriert. Die Proben wurden bis zur weiteren Verwendung bei -20°C aufbewahrt.

### d) Reinigung des Proteins durch reversed phase (r)HPLC

Die zur Trockne eingeengten Wertfraktionen der Cu-Chelat-Chromatographie wurden in 0,1 %iger Trifluoressigsäure(TFA)-Lösung aufgenommen und auf einer C-4 reversed phase Säule (rP304, Biorad, München) weiter gereinigt. Bei diesem Schritt wurden große und kleine Untereinheiten des Proteins getrennt. Die Elution von der Säule wurde durch Anlegen eines linearen Acetonitril-Gradienten (in 0,1 % TFA) von 0 bis 50 % in 90 min bei einem Fluß von 1 ml/min erreicht. Der Verlauf der Elution wurde durch UV-Messung bei 210 nm verfolgt. Unter diesen Bedingungen eluierten die dem Protein zugeordneten Proteinketten zwischen 35 bis 45 % Acetonitril. Die zum Protein gehörenden Proteinbanden wurden fraktioniert und nach Entfernen des Lösungsmittels einem proteolytischen Verdau zur Generierung von Peptiden unterworfen.

### e) Trennung der nach proteolytischer Fragmentierung erhaltenen Destabilase-Peptide mittels reversed phase (r)HPLC

Die nach enzymatischen Verdau mit Trypsin (Sequence Grade, Boehringer Mannheim) bzw. Endoprotease Glu C (Boehringer Mannheim) erhaltenen Peptidgemische wurden mit 0,1 %iger TFA versetzt und auf einer C-18 reversed phase HPLC-Säule (rp 318, Biorad, München) aufgetrennt. Dabei wurde über einen Zeitraum von 2 h ein linearer Gradient von Lösung C (0,1 % TFA in Wasser) nach Lösung D (0,1 % TFA in Acetonitril) angelegt. Bei einem Fluß von 1 ml/min wurde die Elution durch UV-Messung bei 210 nm verfolgt. Die getrennten Peptide wurden fraktioniert und die Aminosäure-Sequenz einzelner Peptide wurde auf einem Peptidsequenzer (Applied Biosystems) ermittelt.

Es wurden die im Sequenzprotokoll angegebenen Sequenzen ermittelt.

### Charakterisierung des Proteins

Nach SDS-Polyacrylamid-Gelelektrophorese und anschließender Silberfärbung sind drei Banden zu erkennen (Low molecular weight marker von Pharmacia): Eine Bande mit einem apparenten Molekulargewicht von 57 000 Da und zwei Banden bei 30 000 Da. Wird das Molekulargewicht durch Molekularsiebchromatographie (TSK G 3000, Puffer: 20 mM Na₂HPO₄ 0,1 M NaCl pH 7,0 Flußrate: 0,7 ml/min) ermittelt, zeigt das Protein ein apparentes Molekulargewicht von ca. 44 000 (± 5000 Da) Da. Die spezifische Aktivität beträgt ca. 580 U/mg. Zur Bestimmung der Aktivität wurde folgender Test verwendet: 50 »l Puffer (5 mM Tris pH 8,4, 0,01 % Tween® 80) 50 »l Farbreagenz (γ-Glutamyl)-p-nitroanilid, 1 mg/ml) wurden mit 50 »l Probe 60 min bei 37°C inkubiert und anschließend die Zunahme der Extinktion bei 405 nm ermittelt. 1 U ist definiert als enzymatische Aktivität, die 1 »Mol γ-Glutamyl-para-Nitroanilid in 1 min bei 37°C umsetzt und dadurch eine Absorptionszunahme bei 405 nm verursacht.

Das natürliche Protein ist ein Glycoprotein, was durch seine Mannose-abhängige Elution von der Concanavalin A-Säule belegt ist.

Protease-Hemmstoffe wie Cystatin C (10 »g/ml), Aprotinin (100 »g/ml) und Benzamidin (10 mM) hemmen die Aktivität nicht. 100 »M Serin zusammen mit 100 »M Borat pH 8,4 hemmen ca. 50 % der enzymatischen Aktivität. Das Enzym wird durch Zugabe von 1 - 5 mM HgCl₂ vollständig gehemmt. Weiterhin hemmen reduziertes und oxidiertes Glutathion das Enzym. Triton® X-100 läßt die Aktivität nahezu unbeeinflußt und kann vor einer Denaturierung durch SDS schützen. Das pH-Optimum liegt zwischen pH 8 und pH 9. Das Enzym ist aber noch bis pH 12 aktiv. Darüber hinaus ist die unspezifische Hydrolyse des Substrats bereits dominant.

### Nachweis der Fibrin-lösenden Wirkung

100 »l Rinderfibrinogen (Miles Nr. 82-0222-4; enthält Faktor XIII) in einer Konzentration von 4 mg/ml in TBS (= 10 mM Tris/HCl pH 7,5, 150 mM NaCl) werden mit 10 »l Rinderthrombin (Sigma Nr. T6634, 5 U/ml in TBS mit 25 mM Kalziumacetat) versetzt und 2 bis 5 h bei 37°C in Eppendorf-Reaktionsgefäßen inkubiert. Dabei entstehen stabile, Faktor XIIIa-quervernetzte Fibringerinnsel.

Diese werden einmal mit 10 mM Natriumphosphat pH 7,5, 150 mM NaCl gewaschen und anschließend mit 200 »l Speichelsekret von Hirudo medicinalis inkubiert (20 h, 37°C). Nach Zugabe von 1 ml 5 %iger Monochloressigsäure wird die freigesetzte Proteinmenge im überstand (OD 280 nm) bestimmt.

### Beispiel 2

### 1. Isolierung von m-RNA

RNA wurde aus ganzen Blutegeln nach Aufschluß in Guanidiniumthiocyanat und anschließender Zentrifugation durch ein CsCl-Kissen gewonnen. Der am 3'-Ende ein polyA enthaltende Teil der RNA (polyA+) wurde über oligo(dT)-Affinitätschromatographie isoliert. Beide Verfahrensschritte wurden gemäß Sambrook et al (et al (1989) "Molecular Cloning" 2nd edition, CSH-Press, Seite 7.19-7.22 und 7.26-7.29 durchgeführt.

### 2. Herstellung von DNA-Sonden

Zur Klonierung von cDNA-Fragmenten mit Hilfe der Polymerase-Kettenreaktion (PCR, für Details dieser Technik s. Sambrook et al, Kapitel 14) wurde von der Peptid-Sequenz
Gly Lys Asp Ile Ile Lys Lys Tyr
ausgegangen. Unter Zugrundelegung des bekannten genetischen Codes läßt sich diese Aminosäuresequenz in eine Nukleinsäuresequenz umschreiben. Wegen der bekannten Degeneration des genetischen Codes sind in machen Positionen mehrere Nukleinsäuren einsetzbar. Damit ergeben sich für die Sequenz des mRNA Strangs folgende 576 Möglichkeiten:
5'GGNAARGAYATHATHAARAARTA3'
Um die Komplexität herabzusetzen, wurden 4 Gruppen von je 144 Oligonukleotiden synthetisiert (316 A-D). Ebenso wurden vom komplementären Strang (cDNA-Strang) 4 Gruppen von je 144 Oligonukleotiden synthetisiert (316 rev A-D).

Als 2. Peptidsequenz wurde das Peptid
Ile Val Gln Glu Ile Gln Ser Glu
verwendet. Nach Umschreiben in den Nukleinsäurecode ergeben sich für den mRNA-Strang folgende 1728 Möglichkeiten:
5'ATHGTNCARGARATHCARTCNGA3'
5'ATHGTNCARGARATHCARAGYGA3'
Diese Sequenz wurde in 12 Gruppen zu je 144 Oligonukleotiden synthetisiert (317 A-L). Ebenso wurden vom komplementären Strang (cDNA-Strang) 12 Gruppen von je 144 Oligonukleotiden (317 rev A-L) synthetisiert.

Die Synthesen wurden mit einem Applied Biosystems DNA-Synthesizer Typ 360A durchgeführt. Die Oligonukleotide wurden nach dem Entfernen der Schutzgruppen gelelektrophoretisch über ein Acrylamid/Harnstoffgel gereinigt.

### 3. Herstellung von cDNA

12 x 3 »g polyA⁺-RNA wurden mit Hilfe eines kommerziell erhältlichen cDNA-Synthesekits (Boehringer Mannheim, Best. Nr. 10 13 882) mit oligo(dT)₁₅ als Starter in einzelsträngige cDNA umgeschrieben. Die Synthese wurde nach 1 Stunde durch Erhitzen auf 95°C (5 min) gestoppt und niedermolekulare Bestandteile über eine "cDNA Spun Column" (Pharmacia Nr. 27-5099) abgetrennt.

### 4. PCR Reaktion

Die Methode der Polymerase-Kettenreaktion (PCR) ist in Sambrook et al, Kapitel 14 beschrieben. Die Methode erlaubt die Vervielfältigung von DNA-Segmenten. Dazu notwendig ist neben der DNA (im vorliegenden Fall die einzelsträngige cDNA) ein Satz komplementärer Oligonukleotide. Dazu muß das eine Oligonukleotid komplementär zum mRNA-Strang, das andere komplementär zum cDNA-Strang sein. Die PCR-Reaktion erlaubt dann, das Segment zwischen den beiden Oligonukleotiden zu vervielfältigen und zu klonieren. Insgesamt wurden 96 PCR-Reaktionen durchgeführt, wobei jeweils die Oligonukleotidgruppen 316 und 317 rev bzw. 316 rev mit 317 kombiniert wurden. Für jede Reaktion wurde 1/10 des cDNA-Ansatzes (0,3 »g RNA) eingesetzt. Die PCR-Reaktion wurde durchgeführt auf einem Gerät der Firma Perkin-Elmer (DNA-Thermo Cycler) mit 40 Zyklen zu je 2 min bei 95°C, 2 min bei 50°C und 2 min bei 72°C.

Die Oligonukleotidmischung 316B
5'GGCAARGAYATHATHAARAARTA3'
kombiniert mit 317 revI
5'TCACTTTGDATYTCYTGNACDAT3'
ergab ein DNA-Fragment der Größe 590 bp. Dieses Fragment konnte aus einem präparativen Agarosegel isoliert und nach dem Anhängen von 5'-Phosphatresten ("Kinasieren") in die SmaI-Schnittstelle des Klonierungsvektors puc 18 kloniert werden. Der Klon wurde Dest-pcr 1 genannt. Die DNA des chimären Plasmids konnte in E. coli (CMK 603) vermehrt und die DNA-Sequenz des Passagiers nach der Sanger-Methode ermittelt werden (Sequenzprotokoll: Sequenz 13). Die hierzu notwendigen Methoden sind in Sambrook et al (1989) ausführlich erläutert.

Die DNA-Sequenz und die dazugehörige Proteinsequenz ist Sequenz 13. Die Sequenz beginnt mit einem Teil von Peptid 11 und endet mit einem Teil von Peptid 1. Die Sequenz von Nukleotid 307 bis 333 kodiert für das Peptid 9.

Sequenzprotokoll

Sequenz 1:
Ile Val Gln Glu Ile Gln Ser Glu Gly Gly Ile Ile Thr Glu Glu Asp
                5                   10                  15
Leu Ala Asn
        18

Sequenz 2:
Phe Val Thr Gly Ala Ser Gly Gly Ser Arg
                5                   10

Sequenz 3:
Leu Leu Gly Phe Glu Val Thr Ala Glu Lys
                5                   10

Sequenz 4
Pro Met Ser Gly Met Val Pro Thr Leu Ile Asp Asp Lys
                5                   10

Sequenz 5
Val Gly Val Cys Val Tyr Lys
                5

Sequenz 6
Phe Val Tyr Thr Gly Thr Val Ala Glu Arg Lys
                5                   10

Sequenz 7
Leu Asn Tyr Ala Pro Ala
                5

Sequenz 8
Ile Ile Glu Ala Phe Ser
                5

Sequenz 9
Leu Phe Gln Pro Thr Ile Asp Leu Leu
                5               10

Sequenz 10
Leu Gln Leu Gly Asp Gln Asn Phe Thr Xaa Val Thr Asp Leu Ile Ser
                5                   10                  15

Sequenz 11
Thr Val Ala Cys Ser Lys Ile Gly Lys Asp Ile Ile Lys Lys Tyr
                5                   10                  15
Sequenz 12
Xab Phe Xac Phe Ala Tyr Ala Gly Pro
                5

Sequenz 13

ATA ATC AAG AAG TAT GGA TCG GTC GTG GAT TCT GCG ATT GCC TCA ATG 48
Ile Ile Lys Lys Tyr Gly Ser Val Val Asp Ser Ala Ile Ala Ser Met
                5                   10                  15

CTC TGC GTT GGA GTG GTC AGT GCT CAC AGC AAT GGA ATT GGA GGA GGT 96
Leu Cys Val Gly Val Val Ser Ala His Ser Asn Gly Ile Gly Gly Gly
            20                  25                  30

CAC GTT GCA ATC ATC TAC GCT AAG CCA AAC AAT GGC ACC AAG AAA GAA 144
His Val Ala Ile Ile Tyr Ala Lys Pro Asn Asn Gly Thr Lys Lys Glu
        35                  40                  45

CTC GTA ACC CTG ATA GCA AGA GAA AGA GCA CCG AAG AAG GCA AAC CAA 192
Leu Val Thr Leu Ile Ala Arg Glu Arg Ala Pro Lys Lys Ala Asn Gln
    50                  55                  60

ACA ATG TTC GCC AGC AGA TCT TCT CTC GCG GGA GGT CTT GCA GCC GGT 240
Thr Met Phe Ala Ser Arg Ser Ser Leu Ala Gly Gly Leu Ala Ala Gly
65                  70                  75                  80

ATT CCT GGA GAA TTA AAG GGA TAT TAC GAA GCT TGG AAA AGA TTT GGA 288
Ile Pro Gly Glu Leu Lys Gly Tyr Tyr Glu Ala Trp Lys Arg Phe Gly
                85                  90                  95

TGG TTG CCA TGG AAA GAC TTG TTT CAA CCA ACA ATC GAT TTG TTG GAA 336
Trp Leu Pro Trp Lys Asp Leu Phe Gln Pro Thr Ile Asp Leu Leu Glu
            100                 105                 110

AAT GGA TAT GTC ATT GAG AAA GCC TTA GCC TCG ACC TTG AAC AAA GAA 384
Asn Gly Tyr Val Ile Glu Lys Ala Leu Ala Ser Thr Leu Asn Lys Glu
        115                 120                 125

ATC GTG GCC TCT CTT GTT CTA AAC ACA AGT CTT GTC AAA AGT TCG TTG 432
Ile Val Ala Ser Leu Val Leu Asn Thr Ser Leu Val Lys Ser Ser Leu
    130                 135                 140

TTG AGG AAA ACC TAT TAT CCA AAT GGC CAA GCG CTC AGG GCT GGT GAC 480
Leu Arg Lys Thr Tyr Tyr Pro Asn Gly Gln Ala Leu Arg Ala Gly Asp
150 155 160 165

ACT TTG AAA GAC CAA AAG TTG GCC GCC ACT TTT AAA AAG ATA GCT CAA 528
Thr Leu Lys Asp Gln Lys Leu Ala Ala Thr Phe Lys Lys Ile Ala Gln
170 175 180

AGT CCA GAG AGT TTT TAC AGC GGT GAT CTT GCA AAA ACG ATC GTT CAG 576
Ser Pro Glu Ser Phe Tyr Ser Gly Asp Leu Ala Lys Thr Ile Val Gln
185 190 195

GAA ATC CAA AGT GA 590
Glu Ile Gln Ser
200

## Patentansprüche

1. Protein, welches die Sequenzen
1. 
Ile Val Gln Glu Ile Gln Ser Glu Gly Gly Ile Ile Thr Glu Glu Asp
Leu Ala Asn,
2. Phe Val Thr Gly Ala Ser Gly Gly Ser Arg,
3. Leu Leu Gly Phe Glu Val Thr Ala Glu Lys,
4. Pro Met Ser Gly Met Val Pro Thr Leu Ile Asp Asp Lys,
5. Val Gly Val Cys Val Tyr Lys,
6. Phe Val Tyr Thr Gly Thr Val Ala Glu Arg Lys,
7. Leu Asn Tyr Ala Pro Ala,
8. Ile Ile Glu Ala Phe Ser,
9. Leu Phe Gln Pro Thr Ile Asp Leu Leu,
10. Leu Gln Leu Gly Asp Gln Asn Phe Thr Xaa Val Thr Asp Leu Ile Ser,
11. Thr Val Ala Cys Ser Lys Ile Gly Lys Asp Ile Ile Lys Lys Tyr,
12. Xab Phe Xac Phe Ala Tyr Ala Gly Pro und
13. Ile Ile Lys Lys Tyr Gly Ser Val Val Asp Ser Ala Ile Ala Ser Met
enthält und ε-(γ-Glutamyl)-Lysin-Bindungen spaltet, sowie dessen Muteine und natürliche Varianten mit einer vergleichbaren Wirkung.

2. Verfahren zur Herstellung der Proteine gemäß Anspruch 1, dadurch gekennzeichnet, daß man sie nach bekannten gentechnischen Verfahren herstellt.

3. DNA kodierend für ein Protein gemäß Anspruch 1.

4. Proteine gemäß Anspruch 1 zur Verwendung bei der Bekämpfung von Krankheiten.

## Claims

1. A protein which contains the sequences
1. 
Ile Val Gln Glu Ile Gln Ser Glu Gly Gly Ile Ile
Thr Glu Glu Asp Leu Ala Asn,
2. Phe Val Thr Gly Ala Ser Gly Gly Ser Arg,
3. Leu Leu Gly Phe Glu Val Thr Ala Glu Lys,
4. 
Pro Met Ser Gly Met Val Pro Thr Leu Ile Asp Asp
Lys,
5. Val Gly Val Cys Val Tyr Lys,
6. Phe Val Tyr Thr Gly Thr Val Ala Glu Arg Lys,
7. Leu Asn Tyr Ala Pro Ala,
8. Ile Ile Glu Ala Phe Ser,
9. Leu Phe Gln Pro Thr Ile Asp Leu Leu,
10. 
Leu Gln Leu Gly Asp Gln Asn Phe Thr Xaa Val Thr
Asp Leu Ile Ser,
11. 
Thr Val Ala Cys Ser Lys Ile Gly Lys Asp Ile Ile
Lys Lys Tyr,
12. Xab Phe Xac Phe Ala Tyr Ala Gly Pro and
13. 
Ile Ile Lys Lys Tyr Gly Ser Val Val Asp Ser Ala
Ile Ala Ser Met
and cleaves ε-(γ-glotamyl)-lysine linkages, and its muteins and natural variants with a comparable action.

2. A process for preparing the proteins as claimed in claim 1, which comprises preparing them by known genetic engineering processes.

3. DNA coding for a protein as claimed in claim 1.

4. A protein as claimed in claim 1 for use for controlling diseases.

## Revendications

1. Protéine qui contient les séquences :
1. 
Ile Val Gln Glu Ile Gln Ser Glu Gly Gly Ile Ile Thr Glu Glu Asp
Leu Ala Asn,
2. Phe Val Thr Gly Ala Ser Gly Gly Ser Arg,
3. Leu Leu Gly Phe Glu Val Thr Ala Glu Lys,
4. Pro Met Ser Gly Met Val Pro Thr Leu Ile Asp Asp Lys,
5. Val Gly Val Cys Val Tyr Lys,
6. Phe Val Tyr Thr Gly Thr Val Ala Glu Arg Lys,
7. Leu Asn Tyr Ala Pro Ala,
8. Ile Ile Glu Ala Phe Ser,
9. Leu Phe Gln Pro Thr Ile Asp Leu Leu,
10. Leu Gln Leu Gly Asp Gln Asn Phe Thr Xaa Val Thr Asp Leu Ile Ser,
11. Thr Val Ala Cys Ser Lys Ile Gly Lys Asp Ile Ile Lys Lys Tyr,
12. Xab Phe Xac Phe Ala Tyr Ala Gly Pro und
13. Ile Ile Lys Lys Tyr Gly Ser Val Val Asp Ser Ala Ile Ala Ser Met
et qui scinde les liaisons ε-(γ-glutamyl)-lysine ainsi que ses mutéines et variantes naturelles avec une activité comparable.

2. Procédé de préparation d'une protéine suivant la revendication 1, caractérisé en ce qu'on la prépare selon des procédés techniques connus.

3. ADN codant pour une protéine suivant la revendication 1.

4. Protéine suivant la revendication 1 pour l'utilisation dans la lutte contre des maladies.
